# EUROPÄISCHE PATENTSCHRIFT

(11) **EP 2 059 195 B1**
(45) Veröffentlichungstag und Bekanntmachung des Hinweises auf die Patenterteilung: **06.04.2011**
(21) Anmeldenummer: 07801250.7
(22) Anmeldetag: 13.08.2007
(51) Int. Cl.: A61F 2/46

(54) **UNIVERSALAUSSCHLÄGER FÜR TOTALENDOPROTHESEN (TEP) DES KNIEGELENKES**
UNIVERSAL EXTRACTOR FOR TOTAL ENDOPROSTHESES (TEP) OF THE KNEE JOINT
EXTRACTEUR UNIVERSEL POUR ENDOPROTHÈSES TOTALES (EPT) DU GENOU

(30) Priorität: 06.09.2006 DE 102006042141
(43) Veröffentlichungstag der Anmeldung: 20.05.2009
(73) Patentinhaber: Arnhold, Christian, 07607 Eisenberg (DE)
(72) Erfinder: ARNHOLD, Michael, 07607 Eisenberg (DE)
(74) Vertreter: Donath, Dirk
(86) Internationale Anmeldenummer: PCT/DE2007/001460
(87) Internationale Veröffentlichungsnummer: WO 2008/028451

(56) Entgegenhaltungen:
- WO-A-93/25164
- WO-A-2007/062103
- DE-A1- 10 013 331
- US-A- 4 601 289
- US-A- 5 732 992

## Beschreibung

Die Erfindung betrifft einen Universalausschläger für Knieendoprothesen gemäß der Gattung der Patentansprüche.

Nur wenige Ausschläger und Spannvorrichtungen sind derzeitig verfügbar; ihre Wirkungsweise ist oft unzweckmäßig.

Die TEP des Kniegelenkes haben durch Fortschritte im Prothesendesign einen fast ebenbürtigen Stellenwert wie die des Hüftgelenkes erreicht. Aber auch die Zahl der Wechseloperationen steigt stetig. Die Grundproblematik ist dabei mit der des Hüftgelenkes vergleichbar.

Auch der Markt der Totalendoprothesenplastiken war vor etwa 20 Jahren von zementierten, gekoppelten Prothesenkomponenten mit Schaft beherrscht. Bei Lockerungen oder Infektionen waren oft ausgeprägte knöcherne Defektsituationen die Folge. Die Entfernung derartiger Prothesen gelang meist ohne Probleme. Bei persistierenden Infekten war eine Amputation keine Seltenheit.

In der Folgezeit eroberten dann die ungekoppelten, meist zementfreien Oberflächenersatzprothesen den Markt, die eine Reihe wesentlicher Vorteile aufweisen. Die Entfernung derartiger Prothesen ist im Revisionsfall nicht unproblematisch. Auch hier gibt es nur wenige technische Lösungen, die im Revisionsfall eine knochenschonende Entfernung des Implantates ermöglichen.

Die wenigen brauchbaren Ausschläger für Knieprothesenkomponenten haben den Nachteil, dass sie keine stabile kraftschlüssige Verankerung an diesen ermöglichen. Ebenso erbringen die Sledge-Hammer meist nur eine unbefriedigende Kraftübertragung bei der Explantation der Gelenkkomponenten.

Im Revisionsfall müssen fest knöchern integrierte zementfreie, seltener auch zementierte Komponenten unter Verwendung diverser Werkzeuge "vorgelockert" werden. Ziel ist eine Reduktion der Verankerungsfläche der Prothesen und damit der Scherkräfte bei der Explantation. Nur so können sekundäre Folgeschäden auf das knöcherne Prothesenlager oder nachteilige Osteotomien vermieden und damit eine Reimplantation einer Revisionsprothese erleichtert werden.

US 5732 992 zeigt die Merkmale des Oberbegriffs von Anspruch 1.

Die Erfindung liegt daher die Aufgabe zu Grunde, einen Universalausschläger für Knieprothesenkomponenten anzugeben, der die Nachteile des Standes der Technik vermeidet. Dazu müssen u.a. folgende Aufgaben gelöst werden:
1. Stabile, orthograde Verankerung des Ausschlägers an Prothesenkomponenten unterschiedlichen Designs und Größe.
2. Optimale Entfaltung der Ausschlagkraft.
3. Wiederverwendbarkeit des Ausschlägers.

Gemäß der Erfindung werden diese Aufgaben durch die kennzeichnenden Merkmale des ersten Patentanspruchs gelöst und durch vorteilhafte Ausgestaltungen gemäß den Unteransprüchen ergänzt.

Der erfindungsgemäße Universalausschläger besteht aus folgenden Baugruppen:
1. Scherenmechanismus mit Spannbacken und Augenschraube mit Rändelmutter.
2. Befestigungsbolzen.
3. T-Stück dessen axialer Schenkel aus einer geschlitzten Stange besteht.

Die stabile Verankerung des Universalausschlägers an der zu entfernenden Prothesenkomponente erfolgt über zwei gekrümmte Spannbacken, die am distalen Ende des Scherenmechanismus angebracht sind. Ihre schaufelartige, näherungsweise V- bis U-förmige Form gestattet auch an gekrümmten Prothesenrändem eine feste, sich selbst zentrierende Fixation. Die sichere Feststellung der Spannbacken ist durch eine am proximalen Ende des Scherenmechanismus gelagerte Augenschraube mit Rändelmutter gewährleistet. Dieser Scherenmechanismus ist über eine Befestigungsachse am axialen Schenkel des T-Stückes mit Ausschlagplatte montiert. Damit ist eine stabile Kopplung zwischen Prothesenkomponente und Ausschlagplatte gesichert, die eine optimale Entfaltung der Ausschlagkraft sicherstellt.

Der Schermechanismus mit den dazugehörigen Spannbacken ist dabei in der Spannweite (dem wirkenden Greifabstand der Spannbacken) einstellbar. Dies erfolgt über je drei in den Schenkeln des Scherenmechanismus eingebrachte Perforationen zur Aufnahme der Befestigungsachsen. Verschiedenste Größen und Geometrien von Prothesenkomponenten sind so stabil und orthograd zentriert verspannbar, die die Funktion des erfindungsgemäßen Universalausschlägers erleichtert.
Die stabile orthograde Verankerung des erfindungsgemäßen Universalausschlägers erfolgt über zwei sich selbst zentrierende Spannbacken, die in die Grenzzone zwischen knöchernem Lager und Prothesenkomponente einbringbar sind. Unabhängig von einer geraden oder gekrümmten Randstruktur einer Prothesenkomponente wird die Selbstzentierung und damit stabile Verankerung durch die ebenfalls gekrümmte Struktur der erfindungsgemäßen Spannbacken erreicht, deren Krümmungsradius jedoch kleiner als der der kleinsten Prothesenkomponente sein muss.
Nach stabiler Fixation des Universalausschlägers an der Prothesenkomponente wird eine optimale Entfaltung der Ausschlagkraft durch die Ausschlagplatte des T-Stückes unter Verwendung eines Metallhammers erzielt.
Nach erfolgreicher Prothesenentfernung kann die Kopplung des Ausschlägers mit dieser problemlos aufgehoben werden. Vorteilhaft ist die stufenlose Spannung und Lösung des Scherenmechanismus.

Die Wiederverwendbarkeit des Universalausschlägers ist durch die Reversibilität der Verankerung und seine Resterilisierbarkeit gegeben.

Die Erfindung wird nachstehend an Hand der schematischen Zeichnungen näher erläutert. Es zeigt:

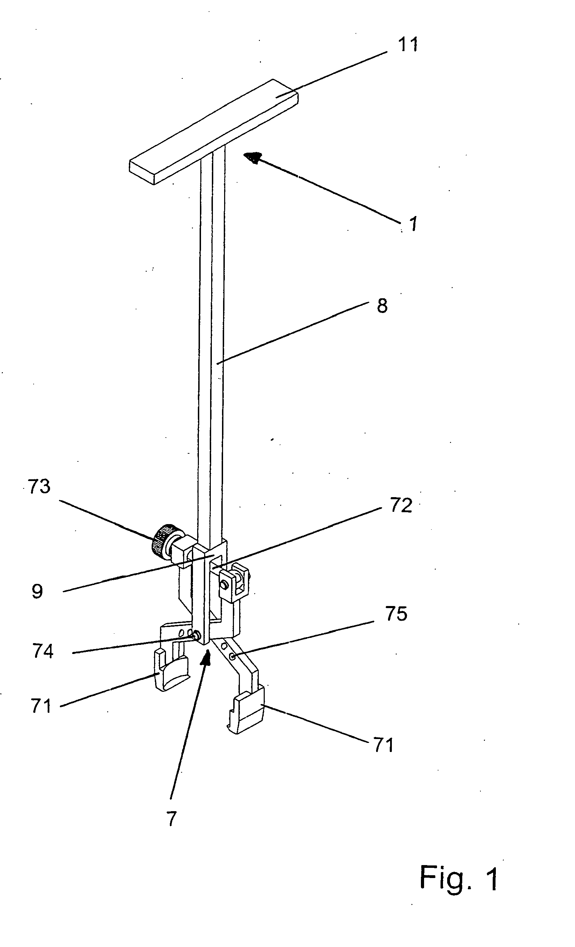

- Fig.1: eine Ausführungsform eines erfindungsgemäßen Universalausschlägers in schematischer räumlicher Darstellung.

Der in Fig. 1 dargestellte Universalausschläger umfasst einen Scherenmechanismus 7 mit Spannbacken 71 sowie eine gelagerte Augenschraube 72 mit Rändelmutter 73. Dieser ist über eine Befestigungsachse 74 mit dem distalen gabelförmigen Ende 9 des Befestigungsschenkels 8 des T-Stückes verbunden. Das proximale Ende des T-Stückes wird durch die Ausschlagplatte 11 gebildet.
Am distalen Ende des Schermechanismus 7 sind die erfindungsgemäßen Spannbacken 71 angebracht. Diese Spannbacken 71 besitzen eine schaufelartige, zum Prothesenrand gerichtete V- bis U-förmige Oberfläche (gekrümmte Kontaktfläche), die die stabile, sich selbst zentrierende Fixation des Ausschlägers am zu entfernenden Prothesenelement in dessen Grenzzonenbereich zum knöchernen Lager gewährleistet. Die Feststellung erfolgt dabei durch die am proximalen Ende des Scherenmechanismus 7 gelagerte Augenschraube 72 mit Rändelmutter 73. Die optimale Spannweiteneinstellung der Spannbacken 71 bei Prothesenelementen unterschiedlicher Größe kann bspw. über eine umsteckbare Befestigungsachse 74 des Scherenmechanismus 7 generiert werden, die in Ausnehmungen 75 gelagert ist. Die Ausnehmungen 75 in den Schenkeln des Scherenmechanismus 7 müssen dabei gleichsinnig sein.

### Bezugszeichenliste

- 1: - T-Stück
- 11: - Ausschlagplatte
- 7: - Scherenmechanismus
- 71: - Spannbacken
- 72: - Augenschraube
- 73: - Rändelmutter
- 74: - Befestigungsachse
- 75: - Ausnehmungen
- 8: - Befestigungsschenkel
- 9: - distales Ende des Befestigungsschenkels 8

## Patentansprüche

1. Universalausschläger für Knieprothesen umfassend ein T-Stück (1) mit einer Ausschlagplatte(11), einen Befestigungsschenkel (8), wobei der Befestigungsschenkel (8) an seinem proximale Ende die Ausschlagplatte (11) trägt und an seinem distalen Ende (9) gabelförmig sowie geschlitzt ist, sowie Spannbacken (71), **dadurch gekennzeichnet, dass** der Universalausschläger einen Scherenmechanismus 7, an dessen freien distalen Enden sich die Spannbacken (71) befinden, umfasst, ferner **dadurch gekennzeichnet, dass**
• die Spannbacken (71) eine schaufelartige, U- bis V-förmige Geometrie sowie einen gekrümmten Rand aufweisen,
• der Befestigungsschenkel (8) den Scherenmechanismus (7) vermittels einer Befestigungsachse (74), um die der Scherenmechanismus (7) bewegbar ist, haltert und
• sich am proximalen Ende des Scherenmechanismus (7) eine gelagerte Augenschraube (72) mit einer Rändelmutter (73) befindet, wobei die Augenschraube (72) verstellbar ist und die Enden des Scherenmechanismus 7 miteinander verbindet, so dass eine Feststellung bzw. Lösung der Spannbacken (71) ermöglicht ist.

2. Universalausschläger gemäß Anspruch 1, **dadurch gekennzeichnet, dass** die Spannbacken (71) einstückiger Bestandteil des Scherenmechanismus (7) sind.

3. Universalansschläger gemäß Anspruch 1, **dadurch gekennzeichnet, dass** der Scherenmechanismus (7) Ausnehmungen (75) aufweist und die Spannweite der Spannbacken (71) durch eine in den Ausnehmungen (75) umsteckbare Befestigungsachse (74) einstellbar ist.

4. Universalausschläger gemäß Anspruch 1, **dadurch gekennzeichnet, dass** im betriebenen Zustand die sichere Feststellung der Spannbacken (74) durch die am proximalen Ende des Scherenmechanismus (7) gelagerte Augenschraube (72) mit Rändelmutter (73) gewährleistet wird.

5. Universalausschläger gemäß Anspruch 1, **dadurch gekennzeichnet, dass** im betriebenen Zustand die Spannbacken (74) selbst zentrierend sind und rotationsstabil ein Prothesenelement fixieren.

## Claims

1. Universal extractor for knee prostheses comprising a T-piece (1) with an extraction plate (11), a securing arm (8) that carries the extraction plate (11) at its proximal end and is fork-shaped and slotted at its distal end (9), and clamping jaws (71), wherein the universal extractor includes a scissor mechanism (7) that is provided with the clamping jaws (71) at its free distal ends, wherein further
• the clamping jaws (71) have a shovel-shaped, U-shaped to V-shaped geometry and a curved edge,
• the securing arm (8) holds the scissor mechanism (7) by means of a securing pin (74) about which the scissor mechanism (7) can move, and
• a supported eyebolt (72) with a knurled nut (73) is provided at the proximal end of the scissor mechanism (7), and said eyebolt (72) is adjustable and connects the ends of the scissor mechanism (7) so as to permit fixation or release of the clamping jaws (71).

2. Universal extractor according to claim 1, wherein the clamping jaws (71) are a one-piece component of the scissor mechanism (7).

3. Universal extractor according to claim 1, wherein the scissor mechanism (7) is provided with through holes (75) and the clamping range of the clamping jaws (71) can be adjusted via a securing pin (74) that can be inserted in or removed from said through holes (75).

4. Universal extractor according to claim 1, wherein the reliable fixation of the clamping jaws (74) in the operating mode is ensured by the eyebolt (72) with knurled nut (73) and said eyebolt is supported at the proximal end of the scissor mechanism (7).

5. Universal extractor according to claim 1, wherein the clamping jaws (74) are self-centering in the operating mode and fix a prosthetic component in a rotation-stable manner.

## Revendications

1. Extracteur universel pour les prothèses du genou contenant une pièce en T (1) avec une plaque d'extraction (11), une nervure de fixation (8), qui porte la plaque d'extraction (11) sur son extrémité proximale et qui est fourchée et fendue sur son extrémité distale (9), ainsi que des mâchoires de serrage (71) est **caractérisé en ce que** l'extracteur universel comprend un mécanisme de ciseaux (7) qui est prévu des mâchoires de serrage (71) sur son libre extrémité distale, en outre **caractérisé en ce que**
• les mâchoires de serrage (71) ont une géométrie en forme de pale, en forme d'U jusqu'à V ainsi qu'un bord voûté,
• la nervure de fixation (8) retient le mécanisme de ciseaux (7) à l'aide d'un axe de fixation (74) autour duquel le mécanisme de ciseaux (7) peut tourner et
• il se trouve un boulon à oeillet (72) sur palier avec un écrou moleté (73) sur l'extrémité proximale du mécanisme de ciseaux (7) et le boulon à oeillet (72) déjà cité peut être ajusté et relie les extrémités du mécanisme de ciseaux (7) de sorte que les mâchoires de serrage (71) puissent être bloquées ou relâchées.

2. L'extracteur universel suivant la revendication 1 est **caractérisé en ce que** les mâchoires de serrage (71) sont un composant en une seule pièce du mécanisme de ciseaux (7).

3. L'extracteur universel suivant la revendication 1 est **caractérisé en ce que** le mécanisme de ciseaux (7) est prévu des creux (75) et que l'écartement des mâchoires de serrage (71) est réglable par un axe de fixation (74) qui peut être enfiché dans les ou enlevé des creux (75).

4. L'extracteur universel suivant la revendication 1 est **caractérisé en ce qu'**une fixation sûr des mâchoires de serrage (74) est assurée en mode opéré grâce au boulon à oeillet (72) sur palier avec l'écrou moleté (73) sur l'extrémité proximale du mécanisme de ciseaux (7).

5. L'extracteur universel suivant la revendication 1 est **caractérisé en ce que** les mâchoires de serrage (74) sont autocentreurs en mode opéré et assurent une fixation stable de l'élément prothétique par rapport à la rotation.
